# EUROPEAN PATENT APPLICATION

(11) **EP 4 650 781 A1**
(43) Date of publication of application: **19.11.2025**
(21) Application number: 24741511.0
(22) Date of filing: 09.01.2024
(51) Int. Cl.: G01N 33/68

(54) **METHOD FOR DIAGNOSING CANCER**

(30) Priority: 10.01.2023 JP 2023001743
(71) Applicant: Miyazaki, Toru, Tokyo 162-8666 (JP)
(72) Inventor: ARAI, Satoko, Tokyo 162-8666 (JP); MIYAZAKI, Toru, Tokyo 162-8666 (JP)
(74) Representative: Engelhard, Markus
(86) International application number: PCT/JP2024/000174
(87) International publication number: WO 2024/150738

(57) **Abstract**

The present invention provides a method for testing for cancer in a subject, including measuring the concentration of an active apoptosis inhibitor of macrophage (AIM) in blood from the subject, wherein it is determined that the subject is potentially suffering from at least one selected from the group consisting of biliary tract cancer and pancreatic cancer, when the concentration of the active AIM in the blood from the subject is higher than that in a healthy subject.

## Description

### [Technical Field]

The present invention relates to a method for diagnosing cancer, by using an apoptosis inhibitor of macrophage.

### [Background Art]

Apoptosis inhibitor of macrophage (AIM; also called CD5 antigen-like (CD5L)) is a blood protein produced by tissue macrophages, which the present inventors identified for the first time as a supporter of macrophage survival. AIM is now recognized as a molecule to induce repair processes in many diseases (Non-Patent Literatures 1 to 3). AIM consists of three cysteine-rich domains (referred to as the SRCR domains), and a unique positively-charged amino-acid cluster is present within the third SRCR domain at the carboxyl terminus. This cluster forms charge-based interactions with dead cells that are strongly negatively charged due to the high-level exposure of phosphatidylserine on their surface (Non-Patent Literatures 1, 2). This association highly enhances the engulfment of dead cells by phagocytes, as AIM is highly internalized by phagocytes via multiple scavenger receptors (Non-Patent Literature 3).

In humans, AIM is generally present while bound to an IgM pentamer. When acute kidney injury or the like occurs, it separates from the IgM pentamer to become free AIM, and binds to dead cells and debris to promote clearance thereof. This mechanism is conserved in many mammals. However, it is known that, in Felids, the binding between AIM and an IgM pentamer is very strong and this mechanism does not function sufficiently (Non-Patent Literature 4). Therefore, dead cell debris in renal tissue is not cleared sufficiently in Felids. As a result, in Felids, dead cell debris accumulated in renal tissue with aging causes renal function to decline and eventually develops severe chronic renal failure, leading to uremia and death.

Considering such background, the present inventors have reported a means for treating or preventing renal diseases in humans and Felids by administering AIM (Patent Literature 1).

However, the relationship between AIM and cancer has not been reported to date.

### [Citation List]

### [Patent Literature]

[Patent Literature 1]
   WO 2015/119253

### [Non Patent Literature]

[Non Patent Literature 1]
   Arai, S. et al., Nat Med. 2016 Feb; 22(2):183-93.
[Non Patent Literature 2]
   Tomita, T. et al., Sci Rep. 2017 Jul 25; 7(1):6450.
[Non Patent Literature 3]
   Arai, S. and Miyazaki, T., Semin Immunopathol. 2018 Nov; 40(6):567-575
[Non Patent Literature 4]
   Sugisawa R. et al., Sci Rep. 2016 Oct 12; 6:35251

### [Summary of Invention]

### [Technical Problem]

The present invention aims to provide a novel means of medical application of AIM.

### [Solution to Problem]

The present inventors measured the concentrations of active AIM and inactive AIM in the blood from patients with various types of cancer and found that (1) the amount of active AIM in the blood significantly increases in patients with certain cancers, and (2) whether a subject is suffering from a certain cancer can be determine by confirming the ratio of active AIM to the total amount of AIM (i.e., the amount of active AIM + inactive AIM) in the blood. Based on such findings, they have conducted further studies and completed the present invention.

That is, the present invention provides the following.
[1] A method for testing for cancer in a subject, comprising
   measuring the concentration of an active apoptosis inhibitor of macrophage (AIM) in blood from the subject,
   wherein it is determined that the subject is potentially suffering from at least one selected from the group consisting of biliary tract cancer and pancreatic cancer, when the concentration of the active AIM in the blood from the subject is higher than that in a healthy subject.
[2] The method of [1], wherein it is determined that the subject is potentially suffering from at least one selected from the group consisting of biliary tract cancer and pancreatic cancer, when the concentration of the active AIM in the blood from the subject is higher than a cutoff value.
[3] A method for testing for cancer in a subject, comprising measuring the concentrations of an active apoptosis inhibitor of macrophage (AIM) and an inactive AIM in blood from the subject, wherein it is determined that the subject is potentially suffering from at least one selected from the group consisting of pancreatic cancer, biliary tract cancer, squamous cell lung cancer, large cell lung cancer, multiple myeloma, non-Hodgkin's lymphoma, and esophageal cancer, when the ratio of the active AIM to the total amount of AIM in the blood from the subject is higher than that in a healthy subject.
[4] The method of [3], wherein it is determined that the subject is potentially suffering from at least one selected from the group consisting of pancreatic cancer, biliary tract cancer, squamous cell lung cancer, large cell lung cancer, multiple myeloma, non-Hodgkin's lymphoma, and esophageal cancer, when the ratio of the active AIM to the total amount of AIM in the blood from the subject is higher than a cutoff value.
[5] The method of any of [1] to [4], wherein the subject is a human.

### [Advantageous Effects of Invention]

According to the present invention, whether a subject is suffering from a certain cancer can be tested with ease.

### [Brief Description of Drawings]

[Fig. 1]

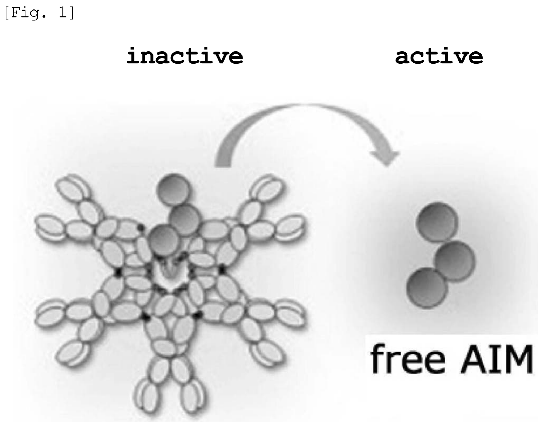

Fig. 1 is a diagram showing the structures of active AIM (free AIM) and inactive AIM.

### [Description of Embodiments]

The present invention is described in detail below.

### 1. Method for testing for cancer - 1

The present invention provides a method for testing for cancer in a subject, including measuring the concentration of an active apoptosis inhibitor of macrophage (AIM) in blood from the subject, wherein it is determined that the subject is potentially suffering from at least one selected from the group consisting of biliary tract cancer and pancreatic cancer, when the concentration of the active AIM in the blood from the subject is higher than that in a healthy subject (hereinafter sometimes referred to as "Test Method 1 of the present invention").

In the Test Method 1 of the present invention, AIM is, for example, in human, a protein having the amino acid sequence shown in SEQ ID NO: 1. AIM includes active form and inactive form. As shown in Fig. 1, inactive AIM exists in the blood in a form bound to an IgM pentamer, and becomes active AIM when released from the IgM pentamer. In the present specification, "active AIM" is also referred to as "free AIM".

The measurement method of the concentration of active AIM present in blood is not particularly limited, and the measurement may be performed using a method known per se. For example, the concentration of active AIM contained in blood can be quantified by preparing serum from blood collected from a subject and performing ELISA method or Western blotting method thereon using an anti-AIM antibody, but is not limited thereto. The anti-AIM antibody can be produced by a method known per se. Alternatively, a commercially available anti-AIM antibody may also be used.

In one embodiment of Method 1 of the present invention, the measurement of the concentration of active AIM present in blood is characterized in that an anti-IgM antibody is not used.

In one embodiment of Test Method 1 of the present invention, whether a subject may be suffering from cancer may also be determined using a cutoff value.

The cutoff value that can be used for the determination is not particularly limited as long as the desired effect can be obtained, and the following cutoff values can be adopted:
[biliary tract cancer]
   generally 1.00 µg/mL or more (e.g., 1.10 µg/mL or more, 1.11 µg/mL or more, 1.12 µg/mL or more, 1.13 µg/mL or more, 1.14 µg/mL or more, 1.15 µg/mL or more, 1.16 µg/mL or more, 1.17 µg/mL or more, 1.18 µg/mL or more, 1.19 µg/mL or more);
   preferably 1.20 µg/mL or more (e.g., 1.21 µg/mL or more, 1.22 µg/mL or more, 1.23 µg/mL or more, 1.24 µg/mL or more);
   further preferably 1.25 µg/mL or more.
[pancreatic cancer]
   generally 1.00 µg/mL or more (e.g., 1.10 µg/mL or more, 1.11 µg/mL or more, 1.12 µg/mL or more, 1.13 µg/mL or more, 1.14 µg/mL or more, 1.15 µg/mL or more, 1.16 µg/mL or more, 1.17 µg/mL or more, 1.18 µg/mL or more, 1.19 µg/mL or more);
   preferably 1.20 µg/mL or more (e.g., 1.21 µg/mL or more, 1.22 µg/mL or more, 1.23 µg/mL or more, 1.24 µg/mL or more, 1.25 µg/mL or more, 1.26 µg/mL or more, 1.27 µg/mL or more, 1.28 µg/mL or more, 1.29 µg/mL or more);
   further preferably 1.30 µg/mL or more (e.g., 1.32 µg/mL or more).

It can be determined that the subject is potentially suffering from at least one selected from the group consisting of biliary tract cancer and pancreatic cancer, when the concentration of the active AIM in the blood from the subject is not less than such cutoff value. In the present specification, the biliary tract cancer may include bile duct cancer and gallbladder cancer.

In Test Method 1 of the present invention, the subject is not particularly limited as long as it is an organism that can suffer from biliary tract cancer or pancreatic cancer. It is generally a primate (e.g., human, chimpanzee, Macaca mulatta, marmoset, etc.), preferably a human.

### 2. Method for testing for cancer - 2

The present invention also provides a method for testing for cancer in a subject, including measuring the concentrations of an active apoptosis inhibitor of macrophage (AIM) and an inactive AIM in blood from the subject, wherein it is determined that the subject is potentially suffering from at least one selected from the group consisting of pancreatic cancer, biliary tract cancer, squamous cell lung cancer, large cell lung cancer, multiple myeloma, non-Hodgkin's lymphoma, and esophageal cancer, when the ratio of the active AIM to the total amount of AIM in the blood from the subject is higher than that in a healthy subject (hereinafter sometimes referred to as "Test Method 2 of the present invention").

AIM, active AIM and measurement method thereof, inactive AIM, and the like in Test Method 2 of the present invention are the same as those explained in Test Method 1 of the present invention.

In Test Method 2 of the present invention, the concentration of inactive AIM, in addition to active AIM, present in blood can be measured.

The measurement method of the concentration of inactive AIM present in blood is not particularly limited, and the measurement may be performed using a method known per se. For example, the concentration of inactive AIM contained in blood can be quantified by performing ELISA method or Western blotting method for inactive AIM, but is not limited thereto.

In one embodiment of Method 2 of the present invention, the measurement of the concentrations of active AIM present and inactive AIM present in blood is characterized in that an anti-IgM antibody is not used.

In one embodiment of Test Method 2 of the present invention, whether a subject may be suffering from cancer may also be determined using a cutoff value.

The cutoff value (%) that can be used for the determination is not particularly limited as long as the desired effect can be obtained, and the following cutoff values can be adopted:
[pancreatic cancer]
   generally 16.0% or more (e.g., 16.1% or more, 16.2% or more, 16.3% or more, 16.4% or more, 16.5% or more, 16.6% or more, 16.7% or more, 16.8% or more, 16.9% or more, 17.0% or more, 17.1% or more, 17.2% or more, 17.3% or more, 17.4% or more, 17.5% or more, 17.6% or more, 17.7% or more, 17.8% or more, 17.9% or more);
   preferably, 18.0% or more (e.g., 18.1% or more, 18.2% or more, 18.3% or more, 18.4% or more, 18.5% or more, 18.6% or more, 18.7% or more, 18.8% or more, 18.9% or more, 19.0% or more, 19.1% or more, 19.2% or more, 19.3% or more, 19.4% or more, 19.5% or more, 19.6% or more, 19.7% or more, 19.8% or more, 19.9% or more);
   further preferably, 20.0% or more (e.g., 20.1% or more, 21.0% or more, 22.0% or more, 23.0% or more, 24.0% or more, 25.0% or more).
[biliary tract cancer]
   generally 20.0% or more (e.g., 20.1% or more, 20.2% or more, 20.3% or more, 20.4% or more, 20.5% or more, 20.6% or more, 20.7% or more, 20.8% or more, 20.9% or more, 21.0% or more, 21.1% or more, 21.2% or more, 21.3% or more, 21.4% or more, 21.5% or more, 21.6% or more, 21.7% or more, 21.8% or more, 21.9% or more);
   preferably, 22.0% or more (e.g., 22.1% or more, 22.2% or more, 22.3% or more, 22.4% or more, 22.5% or more, 22.6% or more, 22.7% or more, 22.8% or more, 22.9% or more, 23.0% or more, 23.1% or more, 23.2% or more, 23.3% or more, 23.4% or more, 23.5% or more, 23.6% or more, 23.7% or more, 23.8% or more, 23.9% or more);
   further preferably, 24.0% or more (e.g., 24.1% or more, 24.2% or more, 24.3% or more, 24.4% or more, 24.5% or more, 24.6% or
   more, 24.7% or more, 24.8% or more, 24.9% or more, 25.0% or more).
[squamous cell lung cancer]
   generally 16.0% or more (e.g., 16.1% or more, 16.2% or more, 16.3% or more, 16.4% or more, 16.5% or more, 16.6% or more, 16.7% or more, 16.8% or more, 16.9% or more, 17.0% or more, 17.1% or more, 17.2% or more, 17.3% or more, 17.4% or more, 17.5% or more, 17.6% or more, 17.7% or more, 17.8% or more, 17.9% or more);
   preferably, 18.0% or more (e.g., 18.1% or more, 18.2% or more, 18.3% or more, 18.4% or more, 18.5% or more, 18.6% or more, 18.7% or more, 18.8% or more, 18.9% or more, 19.0% or more, 19.1% or more, 19.2% or more, 19.3% or more, 19.4% or more, 19.5% or more, 19.6% or more, 19.7% or more, 19.8% or more, 19.9% or more);
   further preferably, 20.0% or more (e.g., 20.1% or more, 21.0% or more, 22.0% or more, 23.0% or more, 24.0% or more, 25.0% or more).
Alternatively, in another embodiment,
   generally 27.0% or more (e.g., 27.1% or more, 27.2% or more, 27.3% or more, 27.4% or more, 27.5% or more, 27.6% or more, 27.7% or more, 27.8% or more, 27.9% or more, 28.0% or more, 28.1% or more, 28.2% or more, 28.3% or more, 28.4% or more, 28.5% or more, 28.6% or more, 28.7% or more, 28.8% or more, 28.9% or more);
   preferably, 29.0% or more (e.g., 29.1% or more, 29.2% or more, 29.3% or more, 29.4% or more, 29.5% or more, 29.6% or more, 29.7% or more, 29.8% or more, 29.9% or more, 30.0% or more, 30.1% or more, 30.2% or more, 30.3% or more, 30.4% or more, 30.5% or more, 30.6% or more, 30.7% or more, 30.8% or more, 30.9% or more);
   further preferably, 31.0% or more (e.g., 31.1% or more).
[large cell lung cancer]
   generally 16.0% or more (e.g., 16.1% or more, 16.2% or more, 16.3% or more, 16.4% or more, 16.5% or more, 16.6% or more, 16.7% or more, 16.8% or more, 16.9% or more, 17.0% or more, 17.1% or more, 17.2% or more, 17.3% or more, 17.4% or more, 17.5% or more, 17.6% or more, 17.7% or more, 17.8% or more, 17.9% or more);
   preferably, 18.0% or more (e.g., 18.1% or more, 18.2% or more, 18.3% or more, 18.4% or more, 18.5% or more, 18.6% or more, 18.7% or more, 18.8% or more, 18.9% or more, 19.0% or more, 19.1% or more, 19.2% or more, 19.3% or more, 19.4% or more, 19.5% or more, 19.6% or more, 19.7% or more, 19.8% or more, 19.9% or more);
   further preferably, 20.0% or more (e.g., 20.5% or more, 21.0% or more, 22.0% or more, 23.0% or more, 24.0% or more, 25.0% or more).
[multiple myeloma]
   generally 16.0% or more (e.g., 16.1% or more, 16.2% or more, 16.3% or more, 16.4% or more, 16.5% or more, 16.6% or more, 16.7% or more, 16.8% or more, 16.9% or more);
   preferably, 17.0% or more (e.g., 17.1% or more, 17.2% or more, 17.3% or more, 17.4% or more, 17.5% or more, 17.6% or more, 17.7% or more, 17.8% or more, 17.9% or more, 18.0% or more, 18.1% or more, 18.2% or more, 18.3% or more, 18.4% or more, 18.5% or more, 18.6% or more, 18.7% or more, 18.8% or more, 18.9% or more);
   further preferably, 19.0% or more (e.g., 19.2% or more, 20.0% or more, 21.0% or more, 22.0% or more, 23.0% or more, 24.0% or more, 25.0% or more).
[non-Hodgkin's lymphoma]
   generally 16.0% or more (e.g., 16.1% or more, 16.2% or more, 16.3% or more, 16.4% or more, 16.5% or more, 16.6% or more, 16.7% or more, 16.8% or more, 16.9% or more);
   preferably, 17.0% or more (e.g., 17.1% or more, 17.2% or more, 17.3% or more, 17.4% or more, 17.5% or more, 17.6% or more, 17.7% or more, 17.8% or more, 17.9% or more, 18.0% or more, 18.1% or more, 18.2% or more, 18.3% or more, 18.4% or more, 18.5% or more, 18.6% or more, 18.7% or more, 18.8% or more, 18.9% or more);
   further preferably, 19.0% or more (e.g., 19.1% or more, 20.0% or more, 21.0% or more, 22.0% or more, 23.0% or more, 24.0% or more, 25.0% or more).
[esophageal cancer]
   generally 16.0% or more (e.g., 16.1% or more, 16.2% or more, 16.3% or more, 16.4% or more, 16.5% or more, 16.6% or more, 16.7% or more, 16.8% or more, 16.9% or more);
   preferably, 17.0% or more (e.g., 17.1% or more, 17.2% or more, 17.3% or more, 17.4% or more, 17.5% or more, 17.6% or more, 17.7% or more, 17.8% or more, 17.9% or more, 18.0% or more, 18.1% or more, 18.2% or more, 18.3% or more, 18.4% or more, 18.5% or more, 18.6% or more, 18.7% or more, 18.8% or more, 18.9% or more);
   further preferably, 19.0% or more (e.g., 19.2% or more, 20.0% or more, 21.0% or more, 22.0% or more, 23.0% or more, 24.0% or more, 25.0% or more).

It can be determined that the subject is potentially suffering from at least one selected from the group consisting of pancreatic cancer, biliary tract cancer, squamous cell lung cancer, large cell lung cancer, multiple myeloma, non-Hodgkin's lymphoma, and esophageal cancer, when the ratio of the active AIM to the total amount of AIM in the blood from the subject is not less than such cutoff value.

In Test Method 2 of the present invention, the subject is not particularly limited as long as it is an organism that can suffer from pancreatic cancer, biliary tract cancer, squamous cell lung cancer, large cell lung cancer, multiple myeloma, non-Hodgkin's lymphoma, or esophageal cancer. It is generally a primate (e.g., human, chimpanzee, Macaca mulatta, marmoset, etc.), preferably a human.

### 3. Treatment method of cancer

The present invention also provides a method for treating cancer in a subject, including a step of determining whether the subject has cancer by using the Test Method 1 or 2 of the present invention, and a step of applying a therapeutic approach for cancer to the subject determined to have cancer (hereinafter sometimes referred to as the "treatment method of the present invention").

In the treatment method of the present invention, the therapeutic approach for cancer is not particularly limited as long as it can treat cancer in a subject. In one embodiment, the therapeutic approach for cancer includes, but are not limited to, anticancer drug treatment, radiation therapy, and surgery. A preferred therapeutic approach for cancer may be an anticancer drug treatment.

Examples of the anticancer drug include, but are not limited to, chemotherapeutic agent, immune checkpoint inhibitor, PARP inhibitor, T cell activation agonist, angiogenesis inhibitor, and the like.

Examples of the chemotherapeutic agent include, but are not limited to, metabolic antagonist, alkaloid, and platinum preparation.

Examples of the metabolic antagonist include, but are not limited to, enocitabine, capecitabine, carmofour, gemcitabine, cytarabine, tegafur, tegafur ·uracil, nelarabine, fluorouracil, fludarabine, pemetrexed, pentostatin, and methotrexate. A particularly preferred example of the metabolic antagonist is gemcitabine.

One example of alkaloid is plant alkaloid. Preferred examples of the plant alkaloid include, but are not limited to, irinotecan, etoposide, sobuzoxane docetaxel, nogitecan, paclitaxel, vinorelbine, vincristine, vindesine, and vinblastine. Preferably, a topoisomerase inhibitor can be mentioned.

Preferred examples of the platinum preparation include, but are not limited to, oxaliplatin, carboplatin, cisplatin, and nedaplatin. Preferably, carboplatin and cisplatin can be mentioned.

Examples of the immune checkpoint inhibitor include, but are not limited to, anti-PD-1 antibody, anti-PD-L1 antibody, anti-CTLA-4 antibody, anti-TIM3 antibody, and anti-LAG3 antibody. Examples of the anti-PD-1 antibody include PembroIizumab (CAS Registry Number: 1374853-91-4), Nivolumab (CAS Registry Number: 946414-94-4), MEDI0680, PDR001, BGB-A317, REGN2810, SHR-1210, PF-0680159I, and various known anti-PD-1 antibodies. Examples of the anti-PD-L1 antibody include AtezoIizumab (CAS Registry Number: 1380723-44-3), Avelumab (CAS Registry Number: 1537032-82-8), Durvalumab (CAS Registry Number: 1428935-60-7), MDX-11 05, and various known anti-PD-L1 antibodies. Examples of the anti-CTLA-4 antibody include Ipilimumab (CAS Registry Number: 477202-00-9), TremeIimumab (CAS Registry Number: 745013-59-6), and various known anti-CTLA-4 antibodies. Examples of the anti-TIM3 antibody include MBG452 and various known anti-TIM3 antibodies. Examples of the anti-LAG3 antibody include BMS-986016s LAG525 and various known anti-LAG3 antibodies. Preferably, anti-PD-L1 antibodies can be mentioned.

Examples of the PARP inhibitor include olaparib, rucaparib, niraparib, veliparib, pamiparib, and talazoparib. Preferably, olaparib can be mentioned.

Examples of the T cell activation agonist include, but are not limited to, agonist antibodies of the TNF receptor superfamily (TNFRSF) or agonist antibodies of co-stimulatory molecules. The target molecule of the "agonist antibodies of the TNF receptor superfamily" is not particularly limited as long as it is a factor that activates cells expressing the TNF receptor superfamily (e.g., T cell, NK cell, etc.). Preferably, it is a factor belonging to the "TNF superfamily" or "TNF receptor superfamily". As the factor belonging to the "TNF superfamily" or "TNF receptor superfamily", a ligand having a trimer structure and a receptor having a trimer structure to which the ligand binds, which contribute to the activation of various immune cells, are known (Nat. Rev. Immunol., 2012,12, 339-51). Examples of the factor belonging to the TNF superfamily or TNF receptor superfamily include CD137, CD137L, CD40, CD40L, 0X40, OX40L, CD27, CD70, HVEM, LIGHT, RANK, RANKL, CD30, CD153, GITR, GITRL, TNFRSF25, and TL1A. Preferably, CD137 can be mentioned. Examples of the CD137 agonist antibody include Urelumab (CAS Registry Number: 934823-49-1), PF-05082566, and various known CD137 agonist antibodies.

A preferred example of the angiogenesis inhibitor is, but not limited to, VEGFR2 antibody. Angiogenesis inhibitors prevent the extensive growth of blood vessels (angiogenesis) necessary for the survival of tumors. For example, angiogenesis, which is promoted by tumor cells to meet the increasing nutritional and oxygen demands, can be blocked by targeting various molecules. Examples of the angiogenesis inhibitor include bevacizumab, sorafenib, everolimus, temsirolimus, and various known angiogenesis inhibitors.

The present invention is explained more specifically in the following Examples; however, the present invention is not limited in any way by these Examples.

### [Example]

### [Example 1] Amount of active AIM and ratio of active AIM to total amount of AIM in blood from healthy subjects or various cancer patients

In order to investigate the relationship between AIM in blood and various cancers, the concentrations of active AIM and total AIM in blood from healthy subjects and patients with various cancers were measured.

### [Measurement method]

The active AIM levels in serum samples were determined by ELISA using mouse anti-human AIM monoclonal antibodies (clones #11 and #12 that were created by the present inventors and specifically recognize active AIM). As for total AIM (i.e., active AIM + inactive AIM), mouse anti-human AIM monoclonal antibodies (clones #6 and #7 that were created by the present inventors and specifically recognize total AIM, available from Immuno-Biological Laboratories Co., Ltd. (Gunma, Japan)) were used. The quantification limits (LOQ; CV<30%) of active AIM and total AIM in the aforementioned ELISA system were 0.17971936 ng/ml and 0.198454825 ng/ml, respectively. Each step was performed using Biomek i7 Automated Workstation (Beckman Coulter) and BioTek 405 LS washer (BioTek Instruments, Agilent Technologies).

Then, an ROC curve was created by calculating the amount of active AIM (µg/mL) in serum or the ratio of active AIM/total AIM (concentration ratio (µg/mL/µg/mL)%) for the presence (patients) or absence (healthy subjects) of various cancers. The cut point (cutoff value) was set to the point on the ROC curve that was closest to (0,1), i.e., the point with perfect sensitivity and specificity.

The measurement results of active AIM (IgM-free AIM) are shown in Table 1, and the measurement results of the ratio of active AIM to the total AIM amount (%F/T) are shown in Table 2. In addition, the results of the numerical analysis of active AIM are shown in Table 3, and the results of the numerical analysis of the ratio of active AIM to the total amount of AIM are shown in Table 4. In Tables 1 and 2, "*" means being statistically significant.

**[Table 1]**

| IgM-free AIM | | | | | |
|---|---|---|---|---|---|
| | **N** | **Mean (pg/mL)** | **S. D. (pg/mL)** | **P value after Holm's correction** | |
| **healthy** | **24** | **0.974018** | **0.504687** | **-** | **-** |
| **multiple myeloma** | **23** | **0.858226** | **0.670989** | **2.034918195** | **-** |
| **Hodgkin's lymphoma** | **24** | **1.033213** | **0.593393** | **0.711376102** | **-** |
| **non-Hodgkin's lymphoma** | **26** | **1.107048** | **0.915545** | **1.572202138** | **-** |
| **breast cancer** | **24** | **0.84379** | **0.600835** | **2.521933225** | **-** |
| **uterine cancer** | **24** | **1.124646** | **0.708901** | **2.807846886** | **-** |
| **cervical cancer** | **24** | **1.242271** | **0.87066** | **2.193736047** | **-** |
| **ovarian cancer** | **24** | **1.24906** | **0.702203** | **1.517518399** | **-** |
| **prostate cancer** | **24** | **1.071742** | **0.504322** | **2.527171835** | **-** |
| **large cell lung cancer** | **24** | **1.805933** | **1.787528** | **0.481235699** | **-** |
| **small cell lung cancer** | **24** | **1.065825** | **0.631807** | **1.161581566** | **-** |
| **squamous cell lung cancer** | **24** | **1.710975** | **1.132716** | **0.09671791** | **-** |
| **lung adenocarcinoma** | **24** | **1.111854** | **0.554404** | **3.350647259** | **-** |
| **esophageal cancer** | **24** | **1.49196** | **1.046823** | **0.503988739** | **-** |
| **gastric cancer** | **23** | **1.128102** | **0.541359** | **3.185644905** | **-** |
| **colon cancer** | **24** | **1.244577** | **1.43081** | **3.115777263** | **-** |
| **biliary tract cancer** | **24** | **1.977279** | **1.395009** | **0.039235372** | ***** |
| **pancreatic cancer** | **24** | **1.733958** | **0.878607** | **0.012489578** | ***** |

**[Table 2]**

| %F/T | | | | | |
|---|---|---|---|---|---|
| | **N** | **Mean (%)** | **S.D. (%)** | **P value after Holm's correction** | |
| **healthy** | **24** | **15.73352** | **6.767517** | **-** | **-** |
| **multiple myeloma** | **23** | **44.72172** | **31.46382** | **0.00321523** | ***** |
| **Hodgkin's lymphoma** | **24** | **21.95669** | **10.87101** | **0.13287355** | **-** |
| **non-Hodgkin's lymphoma** | **26** | **32.54702** | **24.0896** | **0.02242084** | ***** |
| **breast cancer** | **24** | **20.29399** | **14.4178** | **0.33978358** | **-** |
| **uterine cancer** | **24** | **16.81519** | **7.605995** | **0.60514653** | **-** |
| **cervical cancer** | **24** | **24.22413** | **15.91714** | **0.17747059** | **-** |
| **ovarian cancer** | **24** | **22.97419** | **14.14437** | **0.1510638** | **-** |
| **prostate cancer** | **24** | **21.88274** | **9.623314** | **0.12648556** | **-** |
| **large cell lung cancer** | **24** | **28.80134** | **16.28583** | **0.01295994** | ***** |
| **small cell lung cancer** | **24** | **21.88772** | **12.58482** | **0.29267906** | **-** |
| **squamous cell lung cancer** | **24** | **45.51624** | **21.87714** | **1.2018E-05** | ***** |
| **lung adenocarcinoma** | **24** | **21.36873** | **11.04097** | **0.15721202** | **-** |
| **esophageal cancer** | **24** | **31.63899** | **22.51305** | **0.02841703** | ***** |
| **gastric cancer** | **23** | **20.10985** | **9.765546** | **0.24845005** | **-** |
| **colon cancer** | **24** | **28.38142** | **20.86388** | **0.08589055** | **-** |
| **biliary tract cancer** | **24** | **30.56776** | **15.33778** | **0.00199367** | ***** |
| **pancreatic cancer** | **24** | **35.77504** | **19.33111** | **0.00071779** | ***** |

**[Table 3]**

| IgM-free AIM | | | | | |
|---|---|---|---|---|---|
| **vs healthy** | **Cutoff point (µg/mL)** | **sensitivity (%)** | **specificity (%)** | **AUC** | **AUC rank** |
| **multiple myeloma** | **0.787072** | **43.47826** | **45.83333** | **0.360507** | **17** |
| **Hodgkin's lymphoma** | **0.922277** | **45.83333** | **58.33333** | **0.494792** | **13** |
| **non-Hodgkin's lymphoma** | **1.196294** | **42.30769** | **79.16667** | **0.487179** | **14** |
| **breast cancer** | **0.903052** | **33.33333** | **58.33333** | **0.385417** | **16** |
| **uterine cancer** | **1.00728** | **45.83333** | **62.5** | **0.538194** | **12** |
| **cervical cancer** | **1.312882** | **45.83333** | **87.5** | **0.546875** | **10** |
| **ovarian cancer** | **0.900352** | **66.66667** | **58.33333** | **0.637153** | **6** |
| **prostate cancer** | **0.919602** | **62.5** | **58.33333** | **0.569444** | **9** |
| **large cell lung cancer** | **1.087322** | **66.66667** | **70.83333** | **0.722222** | **4** |
| **small cell lung cancer** | **0.899452** | **54.16667** | **58.33333** | **0.539931** | **11** |
| **squamous cell lung cancer** | **1.198544** | **66.66667** | **79.16667** | **0.722222** | **3** |
| **lung adenocarcinoma** | **1.194544** | **50** | **79.16667** | **0.588542** | **7** |
| **esophageal cancer** | **1.054851** | **70.83333** | **66.66667** | **0.654514** | **5** |
| **gastric cancer** | **1.064601** | **52.17391** | **66.66667** | **0.576087** | **8** |
| **colon cancer** | **0.913727** | **45.83333** | **58.33333** | **0.449653** | **15** |
| **biliary tract cancer** | **1.252132** | **70.83333** | **87.5** | **0.723958** | **2** |
| **pancreatic cancer** | **1.31806** | **66.66667** | **87.5** | **0.817708** | **1** |

**[Table 4]**

| %F/T | | | | | |
|---|---|---|---|---|---|
| **vs healthy** | **Cutoff point (%)** | **sensitivity (%)** | **specificity (%)** | **AUC** | **AUC rank** |
| **multiple myeloma** | **19.224305** | **82.6087** | **79.16667** | **0.851449** | **3** |
| **Hodgkin's lymphoma** | **15.080013** | **70.83333** | **62.5** | **0.673611** | **10** |
| **non-Hodgkin's lymphoma** | **19.053011** | **69.23077** | **79.16667** | **0.772436** | **6** |
| **breast cancer** | **17.69185** | **54.17** | **66.67** | **0.565972** | **16** |
| **uterine cancer** | **15.024887** | **54.16667** | **62.5** | **0.532986** | **17** |
| **cervical cancer** | **19.203168** | **62.5** | **79.16667** | **0.633681** | **14** |
| **ovarian cancer** | **19.078541** | **62.5** | **79.16667** | **0.678819** | **9** |
| **prostate cancer** | **18.014737** | **66.66667** | **70.83333** | **0.713542** | **8** |
| **large cell lung cancer** | **20.867367** | **75** | **83.33333** | **0.795139** | **4** |
| **small cell lung cancer** | **15.259786** | **66.66667** | **62.5** | **0.638889** | **13** |
| **squamous cell lung cancer** | **20.2 / 31.05** | **95.83 / 83.33** | **83.33 / 95.83** | **0.946181** | **1** |
| **lung adenocarcinoma** | **15.195762** | **75** | **62.5** | **0.670139** | **12** |
| **esophageal cancer** | **19.255401** | **70.83333** | **79.16667** | **0.729167** | **7** |
| **gastric cancer** | **15.345857** | **60.86957** | **62.5** | **0.626812** | **15** |
| **colon cancer** | **21.146808** | **62.5** | **83.33333** | **0.671875** | **11** |
| **biliary tract cancer** | **24.185724** | **70.83333** | **87.5** | **0.772569** | **5** |
| **pancreatic cancer** | **20.082973** | **87.5** | **83.33333** | **0.888889** | **2** |

As shown in Table 1, it was confirmed that the concentration of active AIM in the blood was significantly higher in patients suffering from biliary tract cancer or pancreatic cancer compared to healthy subjects.

As shown in Table 2, moreover, it was confirmed that the ratio of the amount of active AIM to the total amount of AIM in the blood was significantly higher in patients suffering from pancreatic cancer, biliary tract cancer, squamous cell lung cancer, large cell lung cancer, multiple myeloma, non-Hodgkin's lymphoma, or esophageal cancer, compared to healthy subjects.

### [Industrial Applicability]

According to the present invention, whether a subject is suffering from a certain cancer can be tested with ease. Therefore, the present invention is extremely useful in the medical field such as cancer screening and the like.

This application is based on a patent application No. 2023-001743 filed in Japan (filing date: January 10, 2023), the contents of which are incorporated in full herein.

## Claims

1. A method for testing for cancer in a subject, comprising
measuring the concentration of an active apoptosis inhibitor of macrophage (AIM) in blood from the subject,
wherein it is determined that the subject is potentially suffering from at least one selected from the group consisting of biliary tract cancer and pancreatic cancer, when the concentration of the active AIM in the blood from the subject is higher than that in a healthy subject.

2. The method according to claim 1, wherein it is determined that the subject is potentially suffering from at least one selected from the group consisting of biliary tract cancer and pancreatic cancer, when the concentration of the active AIM in the blood from the subject is higher than a cutoff value.

3. A method for testing for cancer in a subject, comprising measuring the concentrations of an active apoptosis inhibitor of macrophage (AIM) and an inactive AIM in blood from the subject, wherein it is determined that the subject is potentially suffering from at least one selected from the group consisting of pancreatic cancer, biliary tract cancer, squamous cell lung cancer, large cell lung cancer, multiple myeloma, non-Hodgkin's lymphoma, and esophageal cancer, when the ratio of the active AIM to the total amount of AIM in the blood from the subject is higher than that in a healthy subject.

4. The method according to claim 3, wherein it is determined that the subject is potentially suffering from at least one selected from the group consisting of pancreatic cancer, biliary tract cancer, squamous cell lung cancer, large cell lung cancer, multiple myeloma, non-Hodgkin's lymphoma, and esophageal cancer, when the ratio of the active AIM to the total amount of AIM in the blood from the subject is higher than a cutoff value.

5. The method according to any one of claims 1 to 4, wherein the subject is a human.
